(19) Europäisches Patentamt

European Patent Office

Office européen des brevets

(11) **EP 1 496 108 A1**

(12) **EUROPEAN PATENT APPLICATION**
published in accordance with Art. 158(3) EPC

(43) Date of publication:
**12.01.2005 Bulletin 2005/02**

(51) Int Cl.$^7$: **C12N 1/20**

(21) Application number: **03720885.7**

(86) International application number:
**PCT/JP2003/003332**

(22) Date of filing: **19.03.2003**

(87) International publication number:
**WO 2003/080814 (02.10.2003 Gazette 2003/40)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **26.03.2002 JP 2002087215**

(71) Applicants:
- **New Century Fermentation Research Ltd.**
  **Fukuoka-shi, Fukuoka 819-0002 (JP)**
- **SANWA SHURUI CO., LTD**
  **Usa-shi Oita 879-0495 (JP)**
- **Barley Fermentation Technologies Inc.**
  **Usa-shi Oita 879-0467 (JP)**

(72) Inventors:
- **ISHIZAKI, Ayaaki**
  **Fukuoka-shi, Fukuoka 819-0002 (JP)**
- **OMORI, Toshiro,**
  **c/o Barley Fermentation Tech. Inc.**
  **Usa-shi, Oita 879-0467 (JP)**
- **FURUTA, Yoshifumi, c/o Sanwa Shurui Co., Ltd.**
  **Usa-shi, Oita 879-0495 (JP)**
- **UMEMOTO, Yasufumi,**
  **c/o Sanwa Shurui Co., Ltd.**
  **Usa-shi, Oita 879-0495 (JP)**

(74) Representative: **Calamita, Roberto**
**Frank B. Dehn & Co.,**
**European Patent Attorneys,**
**179 Queen Victoria Street**
**London EC4V 4EL (GB)**

(54) **METHOD OF CONTINUOUS CULTURE OF ANAEROBIC BACTERIUM**

(57) In a method for continuous culture of the anaerobic microorganisms that is active cell population is maintained constant, when the fermentation is operating continuously by feeding substrate and alkaline alternatively, residual glucose concentration of the culture liquid can be controlled by feeding the substrate that rate is equal to alkaline consumption rate.

EP 1 496 108 A1

## Description

## Technical Field

The present invention relates to a method for continuous culture of the anaerobic microorganisms.

## Background Art

Among anaerobic microorganisms, there are many industrial important bacteria such as Aceton-Butanol producing microorganism, lactic acid producers, and ethanol producin g bacteria. However, it is not known the process to culture these microorganisms for the modern industrial application except the conventional batch culture. Aceton-Butanol fermentation was the first of the most popular modern fermentation industry until the World War II, however, acetone and butanol are producing by the petrochemical process and no country is using this process. It is well known that lactic acid bacteria are useful microorganism for food industry not only dairy products but also conventional pickles fermentation. Lactic acid production process as organic acid production process is still under development stage unlike glutamic acid fermentation which is the most modern aerobic industrial fermentation among the amino acid and nucleic acid fer mentations. It is known that ethanol fermentation by bacteria has some advantages than yeast process, but no satisfactory process has been introduced to ethanol industry nevertheless many studies involving continuous reactor using immobilized cell process. From current issue about the global tasks such as alternative energy to fossil fuel, reduction of carbon dioxide emission, and environmental hazards by plastic waste, technical attention on the efficient culture methods of anaerobic microorganism. Lactic acid fermentation as a large scale mass production of chemical industry to supply raw material for biodegradable polylactic acid synthesis, and efficient ethanol fermentation process to supply economical sufficient ethanol for gasohol is being paid great attention for an urgent task... However, continuous fermentation process for lactic acid fermentation using *Lactococcus* and *Lactobacillus* and ethanol fermentation using *Zymomorucc* that they are possible to apply industry are not developed. There are many scientific papers describing continuous fermentation for anaerobic microorganisms, but these reports are expressing chemostat culture without proper control of the fermentation kinetics so that continuous fermentation can not last for a long time with adequate control of all fermentation dynamics resulting uneconomical residual glucose concentration with large substrate loss. Thus these results can not be available for industry fermentation process.

The continuous fermentation process which is available for industry must be the process to last long time stationary fermentation state with stable kinetics. for a long time. Substrate feed rate is maintained at constant and residual substrate concentration is low as possible to make minimum loss of the raw material. In continuous fermentation employing aerobic fermentation DO-stat is often used.

In aerobic fermentation, substrate concentration approaches to the critical value (low limit), cell activity decelerates to raise dissolved oxygen level due to decrease of oxygen uptake rate of the cells. Substrate is then fed and fermentation activity will be recovered promptly to last the process.

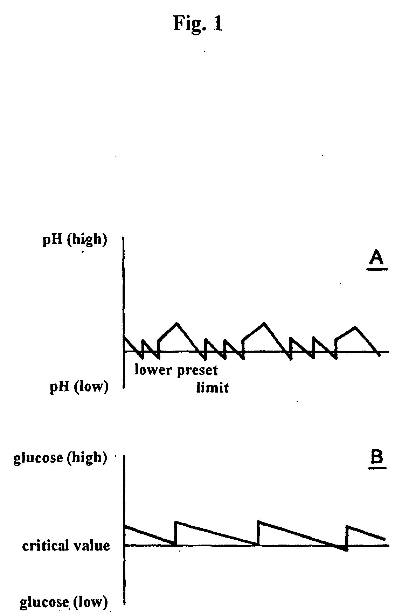

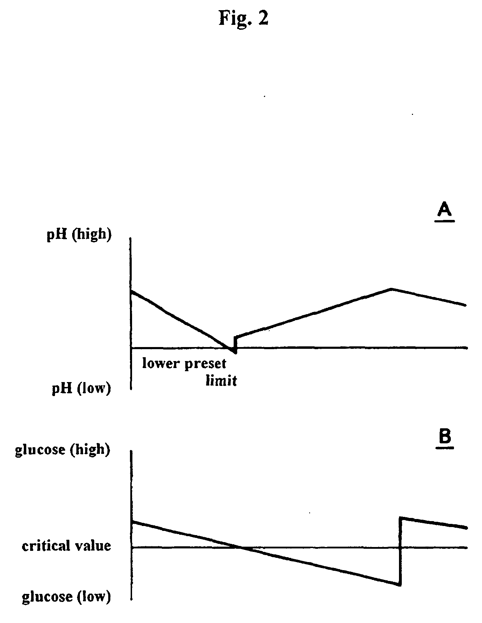

Anaerobic microorganisms do not require oxygen for their metabolism and DO-stat is not possible to use for substrate feed. In another aerobic fermentation, pH-auxostat which is the method for substrate feed using pH up when substrate is used up is used.. Likely in anaerobic fermentation, pH turning to up from down is often observed in ethanol formation as well as lactic acid formation, pH-auxostat may be introduced for anaerobic continuous fermentation. In practice, pH-auxostat in aerobic fermentation was failed. In anaerobic microorganism, cells loss their activities irreversible when pH change turns to rise. As show n in Fig. 1, anaerobic fermentation is possible to last by pH change caused by alternative addition of substrate and alkaline (A) and substrate is fed before residual substrate concentration approached to the critical value (low limit) (B). On the contrary, as shown in Fig. 2, fermentation ceases if substrate is not fed when pH turns to up (A) and residual substrate concentration became lower than the critical value (B). This is special characteristics of anaerobic microorganisms unlike characteristics of aerobic microorganism. Therefore on method has been found for substrate feeding for anaerobic continuous fermentation.

Another problem to solve for continuous anaerobic fermentation is low cell population due to sterile cell formation that is defined by the inventor. Sterile cell is often found in anaerobic bacterium and such cell can not make daughter cell so that such cell growth remains low level of the maximum growth. To increase fermentation rate, high cell population is the effective mean and such culture system can be prepared by cell recycling culture. Cell recycling brings high cell population at the same time serious end product inhibition due to increase of product concentration in the culture broth. Thus cell recycling culture makes serious end product inhibition resulting unstable fermentation kinetics to very poor productivity.

The present invention is aimed to develop new control method for anaerobic continuous fermentation that is available for industry such as high purity L-lactic acid production and biofuel ethanol production. These processes require very high economical efficiency to save minimum production costs.

## Disclosure of Invention

To overcome technical problems mentioned above, the present invention provides following solutions:

1. A method for continuous culture of the anaerobic microorganisms that is active cell population is maintained constant, when the fermentation is operating continuously by feeding substrate and alkaline alternatively, residual glucose concentration of the culture liquid can be controlled by feeding the substrate that rate is equal to alkaline consumption rate.

2. The method for continuous culture of the anaerobic microorganisms according to an above-mentioned invention, wherein the residual glucose concentration is maintained constantly by feeding substrate of molarity that is equal to cumulative consumption molarity of alkaline added in order to control pH of the culture liquid.

3. The method for continuous culture of the anaerobic microorganis ms according to either of above-mentioned inventions, wherein the using diluted alkaline solution forms large dilution effect of culture liquid resulting high specific activity of the microorganisms and high volumetric productivity are maintained.

The present invention has been completed by the following studied conducted by the inventor.

It was not successful the direct control of residual glucose concentration in the culture liquid by feed back control of substrate feed. The suitable sensor and other digital signal to represent the substrate consumption could not be found so that it is found that required amount of glucose supply can be calculated by the amount of alkaline consumed as far as cells activity is maintained. In this system, residual glucose concentration can be controlled at the pre-set level. In this fermentation system, high cell density culture gives high volumetric productivity; however, such culture condition makes high product concentration to develop strong product inhibition. This makes deceleration of specific rate of the fermentation dynamics. Control of residual glucose concentration of such fermentation is difficult. Introducing turbidity control to this fermentation system, cell population becomes under control. This fermentation system makes high product concentration by high cell density but high productivity is still obtained by using diluted alkaline to make large dilution effect. By this manner, end product inhibition in high cell density culture smaller and such fermentation becomes stable with fairly good residual glucose concentration.

Thus the present invention has been completed.

Using the present invention, continuous culture of anaerobic microorganism has been succeeded with fairly good low glucose concentration in the harvest. This process can be applied for industrial production of L-lactic acid. The products from this process are save costs and very high quality with low remained glucose in the final products.

**Brief Description of Drawings**

Fig. 1 shows that in the culture of anaerobic microorganism, pH of the culture liquid approaches to lower preset limit (A), at that time residual glucose concentration decreases to the critical value. Fermentation goes on with healthy, when residual glucose concen tration is still remained above the critical level (B);

Fig. 2 shows that fermentation will cease and can not recover if substrate will be fed after residual glucose concentration become lower than the critical value;

Fig. 3 shows that the schemati c diagram have the mechanical set-up and control system for the present invention.

**Best Mode for Carrying Out the Invention**

In the present invention, cell density of anaerobic microorganism is controlled and continuous culture is conducted by alternative feed of substrate and alkaline. Residual glucose concentration of the spent medium is controlled by the alkaline feed rate using the equation for calculation based on molar equivalent of glucose consumption rate and correction factor. By this control system, raw material loss in the spent medium decreased so that the process can produce high quality product with the minimum cost performance.

Also in the present invention, active cell population is increased by recycling the cell to increase the product concentration resulting strong product inhibition. The productivity of the process can be recovered to high level by high dilution effect performance for the culture system employing diluted alkaline solution for feeding. By these means, specific activ ity of the fermentation can be maintained high and anaerobic continuous fermentation become stable for long time operation.

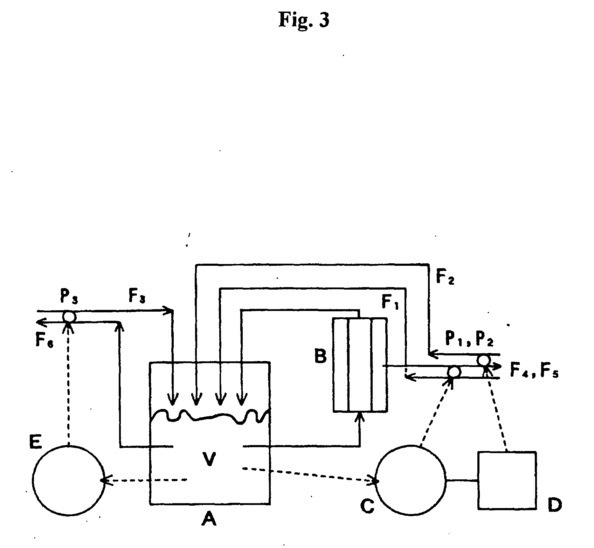

The operation of the system is explaining using the schematic diagram. In Fig. 3., the principle structure of this fermentation system is demonstrated a SFD (Schematic Flow Diagram). In this figure, A: fermentor, B: cross flow filtration for cell separation, C: pH indicator and controller, D: computer for calculation of feed rates of the substrate based on alkaline consumption rate, E: turbidity controller, using laser probe, P1, P2, and P3: peristaltic pumps are indicated. And $F$1: rate of alkaline flow-in to operate pH-stat culture, $F$2: rate of substrate flow-in determined from alkaline consumption rate, $F$3: rate of the medium that does not contain the glucose added for turbidity control flow-in, $F$4 and $F$5: cell free broth exit for feed back control of $F$1 and $F$2 respectively to control fermentation working volume constant, $F$5: broth exit, for feed back control of $F$3 to control the working volume constant

All rates are expressed in a dimension of ml/min. To maintain constant working volume (V) of the fermentor A, the relationship of $F1=F4$ and $F2=F5$ should be remained and at the same time to maintain cell population constant under constant working volume the relationship of $F3=F6$ must be preserved. In this culture system, sum of the rates of stream into the fermentor is $F1+F2+F3$ and sum of bleeding out from the fermentor is $F4+F5+F6$. To maintain constant working volume, the relationship of $F1+F2+F3=F4+F5+F6$ should be established. Thus unlike the conventional reasoning for continuous culture, dilution rate of this continuous fermentation is expressed by the following equation (1).

$$D = \frac{F1+F2+F3}{V} = \frac{F4+F5+F6}{V} \qquad (1)$$

To constant cell population in the fermentor, total growth of the cell in unit time, $\mu XV$ should be equal to cell bleeding out, $XF6$, so that specific growth rate of this fermentation system can be expressed by the relationship $XF6=\mu XV$;.

$$\mu = \frac{F6}{V} \qquad (2)$$

In the conventional reasoning for continuous culture has been expressing by Monod equation which defines that continuous culture will attain the steady state at operation condition of $\mu=D$. The present invention does not agree this theory so that this is a new type continuous fermentation that controlled by new logic. In this new control system, substrate feed rate is given by the function of alkaline feed rate $F1$. If alkaline is diluted to low normality, culture broth will be diluted to increase the productivity due to dilution effect. By this way, end product inhibition becomes smaller and the specific activity of the cells is maintained high so that fermentation becomes steady with fairy good remained glucose concentration.

The following section, describes the examples of the present invention. And o f course, the present invention is not limited by the following examples.

Examples

Example 1:

Strain used is *Lactococcus lactis* IO-1 (JCM7638) which was isolated by the inventor was used Stock culture stored in a deep freezer at -85°C was refreshed in TGC liquid medium (Difco Laboratories, Detroit) and transplanted into 100 ml medium containing in an Erlenmeyer flask for 8 h culture. The medium consisted of 3% of glucose, 0.5% of yeast extract, 0.5% of poly-peptone and 1% g of NaCl and autoclaved for 5 min at 120 °C. Fermentation system employed is the same one shown in Fig. 2. The fermentor is an glassware 1 liter jar with an inner agitation rd driven by magnetic force of gentle agitation 400 rpm. The jar was put in a water bath to which 37°C water was being circulated. An glass electrode for pH measurement (Toa Denpa Go. Tokyo) was installed in the jar and pH of the culture liquid was controlled at the lower limit value (pH 6.0) by feeding of alkaline solution (1N-NaOH). Residual glucose level was periodically determined by an enzymatic glucose analyzer and when glucose concentration reached to 3 g/l continuous culture was started by feeding of substrate the rate of which was calculated based on the rate of alkaline consumption rate. The relationship between glucose demand and alkaline consumption can be considered as the following. That is, the glucose quantity $(G_Q)$ can be written by the following equation (3),

$$G_Q = \frac{fF_1 \times 90}{0.95} + C \qquad (3)$$

where $f$ is a coefficient of normality of 1N-NaOH and $C$ is a term for adjustment of the residual glucose concentration. Glucose demand (g) is equivalent to lactic acid (M.W.=90) formation (g) which corresponds to the rate of 1N alkaline now-in $(F_1)$, however 5 % of the fed glucose should be lost for regeneration of $F_2$. Therefore, the glucose demand is divided by 0.95. Off-set of the control system can be adjusted by the term $C$ according to monitoring residual glucose concentration.

Therefore, to supply glucose quantity which is calculated by an equation (3) using the glucose concentration S g/l, medium feed rate can be calculated by,

$$F_1 = \frac{G_Q}{S} \qquad (4)$$

where S is glucose concentration (g/l) of feed solution.

Continuous culture was conducted by feeding the substrate solution according to the equation (3) and (4). Cell free filtrate from the cross flow ultra filtration was withdrawn The culture broth was withdrawn and recycled back to the fermentor through a cross flow ultra filter (MICROZA PSP103, Asahi-kasei Co., Tokyo). Permeate of the filtration was harvested as lactic acid product solution. Turbidity of the culture broth was determined and controlled at constant cell density by bleeding out the culture broth and feeding non -glucose nutrition solution (yeast extract 0.5%, poly-peptone 0.5% and NaCl 1.0%) as a dilute using a DDC controller (Model LA-300 ASR Co., Tokyo). All streams of the feeding in and bleeding out were synchronized by a peristaltic pump. Three kinds of the feed solutions ($F1$, $F2$ and $F3$) were fed in and two kinds of solution ($F4$ and $F5$, and $F6$) were bleeding out Substrate supply rate is calculated from the alkaline consumption rate for neutralizing lactic acid.

After 12 h batch wise cultivation, residual glucose concentration reached to 3 g/l and the fermentation turned to continuous culture. With substrate feed and cell recycling, cell concentration gradually increased to about 10.5 g/l.

Residual glucose concentration in broth was 4.5 g/l. that are higher than the target value of 2.0 g/l. However, glucose concentration was reduced by manipulating the term C of the equation (3). For 3 h operation, residual glucose concentration has been approached to $2 \pm 0.5$ g/l. Continuous operation lasted for 10 days, 250 h with total dilution rate 0.7 l/h. During continuous operation, average lactic acid concentration of the harvest was 45 g/l, therefore the volumetric productivity of this fermentation was 31.5 g/lh.

From the harvested liquid, L-lactic acid was purified and concentrated to 90 %. Remained glucose in the product was less than 5 % against L-lactic acid and it is satisfactory quality for poly-lactic acid synthesis.

Example 2:

Strain used is *Lactococcus lactis* IO-1 (JCM7638) which was isolated by the inventor was used Stock culture stored in a deep freezer at -85°C was refreshed in TGC liquid medium (Difco Laboratories, Detroit) and transplanted into 100 ml medium containing in an Erlenmeyer flask for 8 h culture. The medium consisted of 3% of glucose, 0.5% of yeast extract, 0.5% of poly-peptone and 1% of NaCl and autodaved for 5 min at 120 °C. Fermentation system employed is the same one used in the example 1. The fermentor is a glassware 1-liter jar with an inner agitation rd driven by magnetic force of gentle agitation at 400 rpm. The jar was put in a water bath to which 37°C water was being circulated. A glass electrode for pH measurement (Toa Denpa Go. Tokyo) was installed in the jar and pH of the culture liquid was controlled at the lower limit value (pH 6.0) by feeding of alkali ne solution (IN-NaOH). The liquid employed for fermentation was enzymatic-hydrolyzed cornstarch diluted to 5 % glucose equivalent Enzyme employed were NOVO Themamyl 120L and Dextrozyme 225/75 L. To the syrup, 1% of corn steep liquor (CSL) was added and p H of the liquid was adjusted before autodaving at 120 °C for 5 min.

Residual glucose level was periodically determined by an enzymatic glucose analyzer and when glucose concentration reached to 1.5 g/l continuous culture was started by feeding of substrate the rate of which was calculated based on the rate of alkaline consumption rate. The relationship between glucose demand and alkaline consumption was given by the equation used in the example 1.

These feeding rates were controlled by the computer. The culture liquid was withdrawn and recyded back to the fermentor through a cross flow ultra filter (NICROZA PSP103, Asahi-kasei Co., Tokyo) and cell free filtrate from the cross flow ultra filtration was withdrawn as harvest solution to recover lactic acid product. To the fermentor a lazar working probe for turbidity determination was installed to control the cell density of the culture broth. The cell density of the culture broth was controlled at constant by bleeding out the culture broth and feeding non-glucose nutrition solution (CSL 1.0%) as a dilute using a DDC controller (Model LA-300 ASR Co., Tokyo). All streams of the feeding in and bleeding out were synchronized by a peristaltic pump. Three kinds of the feed solutions (*F*1, *F*2, and *F*3) were fed in and two kinds of harvest solution (*F*4 and *F*5, and *F*6) were bleeding out. Substrate which is glucose supply rate is calculated from the estimate rate of adding amount of alkaline solution (0.5N-NaOH) for control pH.

After 18 h batch wise cultivation, residual glucose concentration approached to 1.5 g/l and the fermentation shifted to continuous culture. With substrate feed and cell recycling, cell concentration gradually increased to about 123 g/l of DCW.

Residual glucose concentration in culture liquid was 1 g/l. that were lower than the target value of 1.5 g/l. However, glucose concentration was increased by manipulating the term *C*. After 1 h operation, residual glucose concentration has been controlled to $1.5 \pm 0.2$ g/l. Continuous operation lasted for 3 weeks, 525 h with total dilution rate 1.0 l/h. During continuous operation, average lactic acid concentration of the harvest was 40.5 g/l, therefore the volumetric productivity of this fermentation was 40.5 g/lh.

From the harvested liquid, L-lactic acid was purified and concentrated to 90 %. Remained glucose in the product was less than 5 % against L-lactic acid and it is satisfactory quality for poly-lactic acid synthesis.

Example 3:

Microorganism, *Zymomonas mobilis* NRRL-B14023, was used. Stock culture stored in a deep freezer at -85°C was refreshed in YM liquid medium (Difco Laboratories, Detroit) and transplanted into 100 ml medium containing in an Erlenmeyer flask for 8 h culture. The medium consisted of 100 g of glucose, 10 g of yeast extract, 1 g of $KH_2PO_4$, 1 g of $(NH_4)_2P0_4$ and 0.5 g of $MgSO_4 \cdot 7H_2O$ in one liter of deionized water and autodaved for 5 min at 120 °C. Continuous culture was conducted in the fermentation system demonstrated in Fig. 2.

The fermentor is an glassware 1 liter jar with an inner agitator driven by magnetic force of 400 rpm of gentle agitation. The jar was put in a water bath to which 37°C water was being circulated. A glass electrode for pH (Toa Denpa Go. Tokyo) was installed in the jar and pH of the culture broth was controlled at two pre set point, upper limit and lower limit using pH controller. In continuous culture, substrate was fed at upper pH limit and alkaline (0.5N-NaOH) was fed at pH lower limit The culture broth was withdrawn and recycled back to the fermentor through a cross flow ultra filter (MICROZA PSP103, Asahi-kasei Co., Tokyo). Permeate of the filtration was harvested as ethanol product solution. Turbidity of the culture broth was determined and controlled at constant cell density by bleeding out the culture broth and feeding non-glucose nutrition solution as a dilute using a DDC controller (Model LA-300 ASR Co., Tokyo). All streams of the feeding in and bleeding out were synchronized by a peristaltic pump. Substrate supply is calculated from the alkaline consumption for glucose intake by the following equation (5),

$$G_Q = \frac{fF1f_H \times 180}{0.95} + C \qquad (5)$$

where $f_H$ indicates a reciprocal number of ml of 1N-NaOH required for 1 mole (180 g) of glucose intake. Stoichiometry of ethanol fermentation from glucose shows one mole of carbon dioxide release for one mole formation of ethanol. Thus unlike lactic acid fermentation, ethanol yields from glucose theoretically 50 %, not 100%.

Then, if the rate of conversion of ethanol from glucose is expressed as $f_H$, an equation (5) will be given by the same as an equation (1).

In order to maintain constant working volume, accurate volume of cell free filtrate must be withdrawn when substrate solution and alkaline solution was flown in. On the same way, glucose free dilute was fed when the culture broth is bleed out to reduce cell d ensity, so that the working volume of the continuous culture is no change.

Main medium was consisting of 10% of glucose, 1% of yeast extract, 0.5% of $KH_2PO_4$, 0.1% of $(NH_4)_2SO_4$ and 0.05 % of $Mg(SO_4) \cdot 7H_2O$. A 20 ml of the seed culture was transferred to 400 ml of working volume for start up of the culture. pH of the culture was maintained at pH5.5 by feeding alkaline.

At 8 h after start of the main fermentation, residual glucose concentration reached to 1 g/l and continuous culture was introduced with cell recycling. Cell concentration increased and glucose feeding rate proportionally increased, then the cell concentration has reached 7.5g/l as DCW. Residual glucose concentration in broth was slightly high as 1.5 g/l. However, glucose concentration was adjusted by manipulating the term $C$. For 2 h operation, residual glucose concentration has been maintained 1.0±0.3g/l. Continuous culture was lasted for 7 days of 150 h with total dilution rate of 0.5 l/ h and satisfactory low residual glucose level. Ethanol concentration of the harvested liquid was 52 g/l so that ethanol productivity of this continuous culture was 26 g/ l h.

Industrial Applicability

In the present invention, microorganisms employed are not immobilized and maintain cell activity at healthy state at while cells lost activity and sterile cells allow to washout from the reactor and to regenerate the healthy cells. Thus this reactor will be consisting of the active and healthy cells. Such bioreactor gives small deviation of the fermentat ion kinetics by introducing turbidity control for cell population. The system can easily operate continuous mode by feeding substrate and bleeding out the culture broth. Dilution rate of this system gives by the total volume of the feeding solution including alkaline. Diluted alkaline solution makes large dilution effect resulting small end product inhibition. When cell density become high, product concentration increases and represses the product formation rate. However in such case, product formation rate can be maintained high by use of diluted alkaline. None of this type of bioreactor is ever known. Rate of the bioprocess reaction of this new reactor is large as petrochemical process in continuous operation, so that volumetric productivity of this bioprocess gives a few ten times of that of batch wise operation.

**Claims**

1. A method for continuous culture of the anaerobic microorganisms that is active cell population is maintained constant, when the fermentation is operating continuously by feeding substrate and alkaline alternatively, residual glucose concentration of the culture liquid can be controlled by feeding the substrate that rate is equal to alkaline consumption rate.

2. The method for continuous culture of the anaerobic microorganisms acco rding to Claim 1, wherein the residual glucose concentration is maintained constancy by feeding substrate of molarity that is equal to cumulative consumption molarity of alkaline added in order to control pH of the culture liquid.

3. The method for continuous culture of the anaerobic microorganisms according to Claim 1 or 2, wherein the using diluted alkaline solution forms large dilution effect of culture liquid resulting high specific activity of the microorganisms and high volumetric productivity are maintained.

Fig. 1

pH (high)

A

lower preset
limit

pH (low)

glucose (high)

B

critical value

glucose (low)

# Fig. 2

**A**

pH (high)

lower preset
limit

pH (low)

**B**

glucose (high)

critical value

glucose (low)

Fig. 3

## INTERNATIONAL SEARCH REPORT

| International application No. |
| --- |
| PCT/JP03/03332 |

**A. CLASSIFICATION OF SUBJECT MATTER**
Int.Cl⁷ C12N1/20

According to International Patent Classification (IPC) or to both national classification and IPC

**B. FIELDS SEARCHED**

Minimum documentation searched (classification system followed by classification symbols)
Int.Cl⁷ C12N1/00-1/38, C12M1/00-1/42, C12P7/06, C12P7/40

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 58-98085 A (Hitachi, Ltd.)., 10 June, 1983 (10.06.83), (Family: none) | 1-3 |
| Y | JP 53-75033 A (Matsushita Electric Industrial Co., Ltd.), 04 July, 1978 (04.07.78), (Family: none) | 1-3 |
| Y | JP 7-177876 A (Yakult Honsha Co., Ltd.), 18 July, 1995 (18.07.95), (Family: none) | 1-3 |
| A | JP 9-65873 A (Oriental Yeast Co., Ltd.), 11 March, 1997 (11.03.97), (Family: none) | 1-3 |

☒ Further documents are listed in the continuation of Box C. ☐ See patent family annex.

| | |
| --- | --- |
| * Special categories of cited documents: <br> "A" document defining the general state of the art which is not considered to be of particular relevance <br> "E" earlier document but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |
| Date of the actual completion of the international search <br> 24 April, 2003 (24.04.03) | Date of mailing of the international search report <br> 13 May, 2003 (13.05.03) |
| Name and mailing address of the ISA/ <br> Japanese Patent Office | Authorized officer |
| Facsimile No. | Telephone No. |

Form PCT/ISA/210 (second sheet) (July 1998)

# EP 1 496 108 A1

## INTERNATIONAL SEARCH REPORT

International application No.

PCT/JP03/03332

| | C (Continuation). DOCUMENTS CONSIDERED TO BE RELEVANT | |
|---|---|---|
| Category* | Citation of document, with indication, where appropriate, of the relevant passages | Relevant to claim No. |
| A | EP 458370 A (Daicel Chemical Industries), 27 November, 1991 (27.11.91), & DE 3650395 A & JP 61-293388 A & US 5466588 A | 1-3 |
| A | JP 61-124374 A (Matsushita Electric Industrial Co., Ltd.), 12 June, 1986 (12.06.86), (Family: none) | 1-3 |
| A | JP 8-252088 A (Nippoh Chemicals Co., Ltd.), 01 October, 1996 (01.10.96), (Family: none) | 1-3 |

Form PCT/ISA/210 (continuation of second sheet) (July 1998)

11